# EUROPEAN PATENT SPECIFICATION

(11) **EP 2 432 423 B1**
(45) Date of publication and mention of the grant of the patent: **21.09.2016**
(21) Application number: 10718301.4
(22) Date of filing: 07.04.2010
(51) Int. Cl.: A61F 2/06

(54) **IMPROVED VASCULAR PROSTHESIS AND RELEVANT METHOD FOR REALISING THEREOF**
VERBESSERTE GEFÄSSPROTHESE UND RELEVANTES VERFAHREN ZU IHRER FREISETZUNG
PROTHÈSE VASCULAIRE AMÉLIORÉE ET PROCÉDÉ AFFÉRENT DESTINÉ À LA RÉALISATION DE CELLE-CI

(30) Priority: 21.05.2009 IT RN20090023; 25.11.2009 IT RM20090620
(43) Date of publication of application: 28.03.2012
(73) Proprietor: Gessaroli, Massimiliano, 47841 Cattolica (IT)
(72) Inventor: Gessaroli, Massimiliano, 47841 Cattolica (IT)
(74) Representative: Tiburzi, Andrea
(86) International application number: PCT/IT2010/000146
(87) International publication number: WO 2010/134108

(56) References cited:
- EP-A2- 0 955 019
- WO-A1-01/52776
- US-A- 3 818 511
- US-A1- 2008 082 161
- US-A1- 2008 177 379

## Description

The present invention relates to an improved vascular prosthesis and relevant method for realising thereof.

More specifically, the invention concerns a vascular prosthesis that can better conform to the pulsation of blood vessels, thus reducing risk that the same can detach from the blood vessel to which it is fixed, and mainly that do not generate, where it is fixed to the blood vessel, a lumen narrowing, as it always occurs with present prosthesis, thus losing its functionality.

As it is well known, prosthesis for realising bypass or for haemodialysis have been employed in vascular surgery.

Present synthetic vascular prosthesis are mainly employed for replacing obstructed or expanded arterial peripheral vessels (in case of aneurysms) or to realise the so called prosthetic vascular access for haemodialysis. Said prostheses are mainly comprised of ePTFE (expanded polyfluorotetraethylene). Said vascular prosthesis are realised with different shapes. Particularly, they can be rectilinear or conical, with different thicknesses (standard or thin), with or without an enlarged end portion (also known as cap), with the wall impregnated or otherwise bond with heparin.

However, although such an evolution occurred during the last years, present synthetic prosthesis are in any case characterised by a high probability of clogging, mostly due to progressive distal anastomosis (term indicating the point where two vessels are communicated or a prosthesis is connected with a vessel) narrowing (phenomenon also known as stenosis) in peripheral bypass or of venous anastomosis in prosthetic vascular accesses.

Said vessel narrowing is caused by an enormous proliferation of smooth muscular fibre cells, due to increase of activity of fibroblasts present in the vessel wall of the juxta-anostomotic portion (i.e. the portion close to the anastomosis), which are stressed by wall shear stress, i.e. stress to which vessel wall is subjected due to the different elastic response of the same with respect to the prosthetic wall (different compliance) to the haematic pulsating wave.

Many studies, both histopathologic and biomechanical studies, have demonstrated that main reasons of said anostomotic hyperplasia comprise surgical trauma, biocompatibility of synthetic prosthesis, as well as different local mechanical stresses on vessel wall, such as endo-lumen hypertension, flow vortex of some areas and vibrations of the vessel walls caused by flow within connection between vessels (juxta-anostomotic connection).

A first attempt for reducing said phenomenons has been introduced by W.L. Gore & Associates Inc., which produced a prosthesis made up of longitudinally stretchable ePTFE caused by cardiac sistodiastolic mechanical stress, thus trying reproducing kinetic of an arterial or venous vessel. Said solution, even inducing a small improvement of life of said prosthesis, has not solved the above mentioned problems. It is further possible explaining that this result by the fact that, during the modification of the arterial pressure wave during the cardiac sisto-diastolic phases, vessels generically respond with transverse rather than longitudinal pulsation and expansion, and thus also this kind of prosthesis has a remarkable different compliance with respect to original vessels.

A further attempt according to the prior art is described in US patent US 6,589,278, wherein it is suggested adding a cup to the synthetic prosthesis, said cup being comprised of autologous venous material of the same patient, interposed between the prosthetic segment and the vessel to be anastomised. However, in this case too results obtained, although better than those obtained without venous cup, do not fully solve the above mentioned problems, since shear stress reproduces between prosthesis and the same venous cup.

Other authors attempted reducing incidence of anostomotic hyperplasia, directly creating an ePTFE prosthesis, enlarged at one end (cup) in order to improve flow hemodynamic at the anostomotic level, but also in this case the expected result was not conform to expectations since, in any case, remains an elasticity biomechanical difference of vessel with respect to prosthesis, which, under the systolic wave, determines a continuous micro trauma at the sutra level on the same vessel. US3818511 describes a medical prosthesis for ducts or conduits according to the preamble of claim 1.

Therefore, object of the present invention is that of suggesting an improved vascular prosthesis able limiting phenomenon of anostomotic myointimal hyperplasia of vessels in contact point between prosthesis and vessel, thus obtaining a longer life of the same prosthesis.

These and other results are obtained according to the invention providing a prosthesis that can absorb stresses due to arterial pressure waves during sisto-diastolic cardiac phases in suture point of the same prosthesis on vessel wall also along a transverse direction.

It is therefore specific object of the present invention a tubular shaped vascular prosthesis, mainly extending along the longitudinal dimension, which comprises a main portion, having a first and a second end, and at least an end portion having an elasticity along its transverse direction with respect to its longitudinal dimension, said end portion being coupled with one of said first or second end of said main portion and can be coupled to the wall of a blood vessel.

Always according to the invention, said prosthesis can comprise two end portions, respectively coupled with said first and second end.

Still according to the invention, said at least one end portion and said main portion can be realised as a single piece.

Furthermore, according to the invention, said at least one end portion can be coupled to the relevant end of said main portion for suture and/or gluing and/or welding, particularly heat welding.

Advantageously, according to the invention, said main portion can have elasticity along said longitudinal direction.

Always according to the invention, said main portion can have a transverse elasticity with respect to said longitudinal direction.

Still according to the invention, said prosthesis can be of the type suitable for peripheral bypass and/or haemodialysis.

Furthermore, according to the invention, said prosthesis can be comprised of expanded polytetrafluoroetyhlene, of the stretch, standard and/or intering and/or Propaten type and/or of any other elastic prosthetic material.

Advantageously, according to the invention, said at least one end portion can be chosen on the basis of the following groups: rectilinear portion; conical portion; rectilinear bevelled portion; conical bevelled portion.

It is further object of the present invention a method for realising a vascular prosthesis, comprising the following steps:
(a) providing a main vascular prosthesis;
(b) providing a portion of a further vascular prosthesis having elasticity along said longitudinal dimension all along its extension;
(c) longitudinally sectioning said further portion of vascular prosthesis of said step (b);
(d) transversely folding and coupling said portion of sectioned vascular prosthesis of said step (c); and
(e) coupling said portion of said further vascular prosthesis with said main vascular prosthesis.

Always according to the invention, in said step (d), said portion of a further vascular prosthesis is fold and transversely sutured, so as to obtain said end portion chosen on the basis of one of the following groups: rectilinear portion; conical portion; rectilinear bevelled portion; conical bevelled portion.

Still according to the invention, coupling in said steps (d) and/or /e) occurs by suture and/or gluing and/or welding, particularly heat welding.

Advantageously, according to the invention, said main vascular prosthesis can have elasticity along its longitudinal direction and said portion of said vascular prosthesis portion is obtained by a section of a segment of said main vascular prosthesis.

Present invention will be now described for illustrative but not limitative purposes according to its preferred embodiments, with particular reference to the figures of the enclosed drawings, wherein:
figure 1 shows a vascular prosthesis for peripheral bypass according to the present invention;
figure 2 shows vascular prosthesis of figure 1 connecting two arterial vessels;
figure 3 shows a vascular prosthesis for haemodialysis according to the present invention;
figure 4 shows vascular prosthesis of figure 1 connecting an arterial vessel and a venous vessel;
figures 5a - 5d show a plurality of embodiments of a part of prosthesis according to the invention; and
figure 6 shows steps of realisation of a vascular prosthesis according to the present invention.

Similar parts in various figures will be indicated by the same reference numbers.

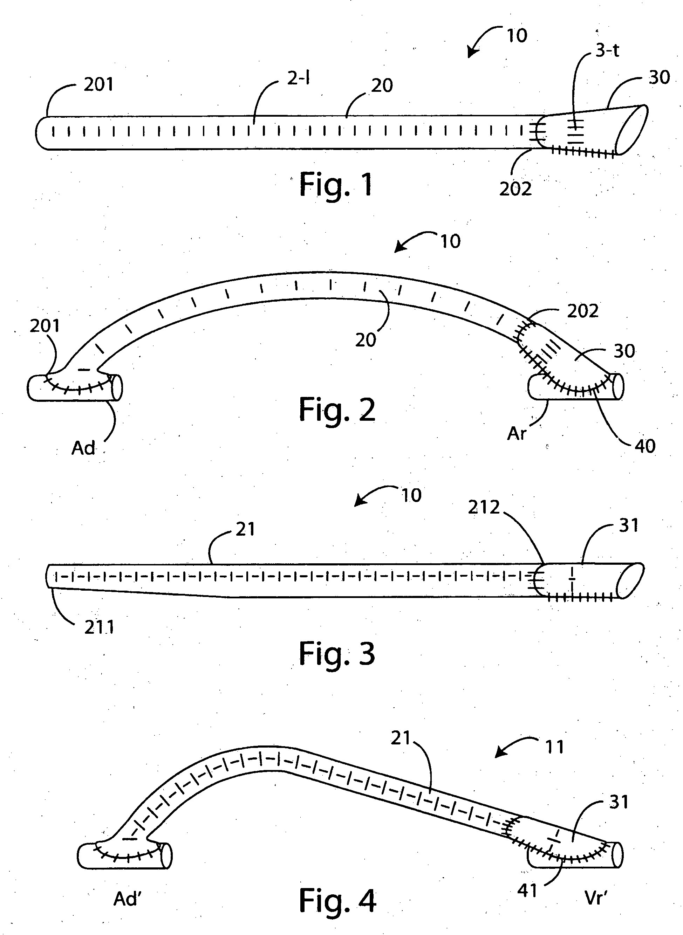

Making reference to figure 1, it is observed a vascular prosthesis 10 for peripheral bypass according to the invention, comprising a main portion 20, extending longitudinally, a first and a second ends 201 and 202. Said main portion 20 is graphically defined in the figure by longitudinal lines 2-I, indicating longitudinal direction according to which said vascular prosthesis 10 portion can be elastically extended.

Vascular prosthesis 10 also comprises an end portion 30, fixed at the second end 202 of said main portion 20. End portion 30 is graphically identified by transverse lines 3-t, showing that said end portion 30 is elastic according to a direction which is transverse with respect to the direction of the main portion 20, directed along its longitudinal direction. Said end portion 30 can be coupled to the wall of a blood vessel.

Figure 2 shows connection of two arterial vessels by vascular prosthesis 10 for peripheral bypass described in the above. Particularly, vascular prosthesis 10 connects a donor artery Ad and a receiving artery Ar. Longitudinal elasticity permits to main portion 20 of prosthesis longitudinally elongating under cardiac sisto-diastolic mechanic stresses generated by donor artery Ad. Instead, end portion 30 permits limiting possible micro-traumas along suture line 40 connecting between end portion 30 of vascular prosthesis 1 and receiving vein Vr wall. Said micro traumas are always due to cardiac sisto-diastolic mechanical stresses, creating a standard transverse (or radial) pulsating movement of said receiving artery Ar thanks to its transverse elasticity, end portion 30 being able to conform its cross-section, thus following said alternate movement.

Figure 3 shows a vascular prosthesis 11 for haemodialysis always comprising a main portion 21, wherein first end 211 has a narrowing or tapering, just for haemodialysis, while at the second end 212 it is coupled an end portion 31, having an elasticity which is transverse with respect to elasticity of main portion 21. In the present embodiment, said end portion 31 is coupled with said second end 121 of said main portion 21 by suture.

Figure 4 shows connection of a donor artery Ad' and a receiving vein Vr' by vascular prosthesis 11 for haemodialysis as described in the above. As it can be observed, in this case too, said end portion 31 is surgically coupled with an opening obtained on the wall of said receiving vein Vr' along the suture line 41. Operation of vascular prosthesis 11 it is similar to the one described for vascular prosthesis 10.

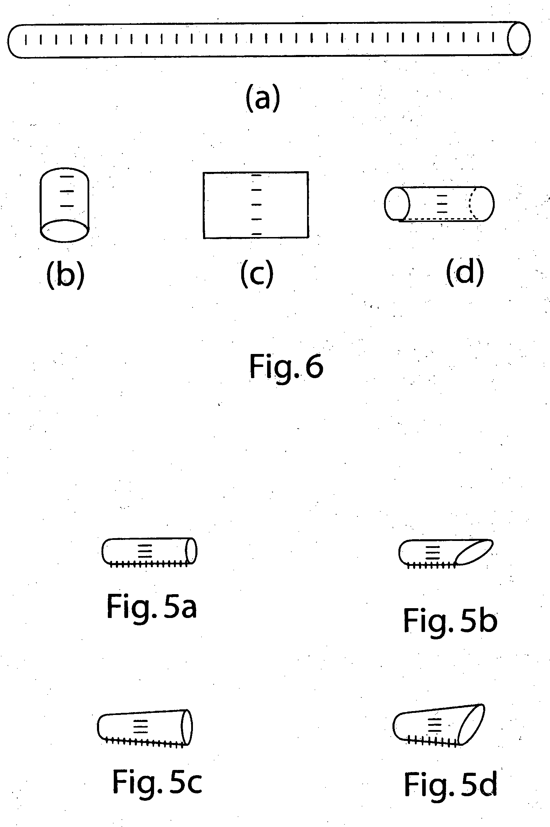

Figures 5a - 5d show different embodiments of end portion 30 or 31 of vascular prosthesis 10 or 11. More specifically:
- figure 5a shows a rectilinear-type end portion 30 or 31;
- figure 5b shows a rectilinear bevelled-type end portion 30 or 31;
- figure 5c shows a conical-type end portion 30 or 31;
- figure 5d shows a conical bevelled-type end portion 30 or 31.

Finally, figure 6 shows steps of a method for realising a vascular prosthesis 1 according to the invention, namely:
(a) providing a main vascular prosthesis, for example for peripheral bypass or for haemodialysis, having a longitudinal elasticity all along its extension;
(b) providing a portion of a further vascular prosthesis having elasticity along said longitudinal dimension all along its extension;
(c) longitudinally sectioning said further portion of vascular prosthesis;
(d) transversely folding and suturing said portion of said further vascular prosthesis; and
(e) suturing said portion of said further vascular prosthesis with said main vascular prosthesis.

Different modifications of the prosthesis according to the invention can be taken into consideration, such as those wherein said main portion 20 does not have elastic properties, or it has an elasticity transversal with respect to its longitudinal direction.

Above method is an hand-craft assembling of a prosthesis having a longitudinal elasticity, for example using standard stretch PTFE prosthesis manufactured by W.L. Gore, in its standard, Intering (with PTFE intraparietal reinforcing rings), Propaten (PTFE with covalent bond with Heparin), with an end portion (cap) comprised of a segment of the same kind of prosthesis. However, it is possible realising prosthesis also by a material different with respect to PTFE or like, with transverse elasticity, which is longitudinally sectioned and then knit transversely, having at the end a transverse elasticity (synthetic cap for vascular prosthesis) that can be compared with the one of a vessel.

Solution providing a direct knitting of tissue in order to generate cap and its coupling to prosthesis when it is the method is available, but as a further modification, it is possible employing different methods such as gluing or welding, particularly heat welding. Said end portion or cap can be realised in such a way to make a rectilinear prolongation of prosthesis or it can be conical.

Furthermore, it must be taken into consideration that vascular prosthesis 10 or 11 thus assembled can be used in vascular fittings for haemodialysis, anatomising part of cap or end portion by efferent nein or it can be used in peripheral bypass anatomising part of the cap with receiving distal artery.

Present invention can also be integrally realised to one or both transverse elasticity ends.

In a further embodiment, vascular prosthesis 10 and 11 according to the invention can be manufactured and realised as a single piece.

The present invention has been described for illustrative, but not limitative purposes, according to its preferred embodiments, but it is to be understood that variations and/or modifications can be introduced by those skilled in the art without departing from the relevant scope, as defined in the enclosed claims.

## Claims

1. Tubular shaped vascular prosthesis (10; 11), mainly extending along the longitudinal dimension, said vascular prosthesis (10; 11) comprising
a main portion (20; 21), having a first (201; 201) and a second end (202; 212), and
at least one end portion (30; 31) extending along said longitudinal dimension
said at least one end portion (30; 31) is coupled with one of said first (201; 211) or second end (202; 212) of said main portion (20; 21), and can be coupled to the wall of a peripheral venous or arterial blood vessel, and has a cylindrical or troncoconical shape,
so that said at least one end portion (30; 31) is capable to conform its cross-section to the alternate movement that can be created by a transverse or radial pulsating movement of said vessel to which it can be coupleable, **characterized in that** said at least one end portion (30;31) has an elasticity along its transverse direction with respect to said longitudinal dimension and said main portion (20;21) has an elasticity along said longitudinal direction.

2. Vascular prosthesis (10; 11) according to claim 1, **characterised in that** it comprises two end portions (30; 31), respectively coupled with said first (201; 211) and second end (202; 212).

3. Vascular prosthesis (10; 11) according to one of the preceding claims, **characterised in that** said at least one end portion (30; 31) and said main portion (20; 21) are realised as a single piece.

4. Vascular prosthesis (10; 11) according to one of the preceding claims 1 - 2, **characterised in that** said at least one end portion (30; 31) is coupled to the relevant end of said main portion (20; 21) for suture and/or gluing and/or welding, particularly heat welding.

5. Vascular prosthesis (10; 11) according to one of the preceding claims 1 - 4, **characterised in that** said main portion (20; 21) has a transverse elasticity with respect to said longitudinal direction.

6. Vascular prosthesis (10; 11) according to one of the preceding claims, **characterised in that** said it is of the type suitable for peripheral bypass and/or haemodialysis.

7. Vascular prosthesis (10; 11) according to one of the preceding claims, **characterised in that** said it is comprised of expanded polytetrafluoroetyhlene (PTFE), of the stretch, standard and/or intering and/or Propaten type and/or of any other elastic prosthetic material.

8. Vascular prosthesis (10; 11) according to one of the preceding claims, **characterised in that** said at least one end portion (30; 31) is chosen on the basis of the following groups:
- rectilinear portion;
- conical portion;
- rectilinear bevelled portion;
- conical bevelled portion.

9. Method for realising a vascular prosthesis (10; 11) as defined in claims 1 - 8, comprising the following steps:
(a) providing a main vascular prosthesis;
(b) providing a portion of a further vascular prosthesis having elasticity along said longitudinal dimension all along its extension;
(c) longitudinally sectioning said further portion of vascular prosthesis of said step (b);
(d) transversely folding and coupling said portion of sectioned vascular prosthesis of said step (c); and
(e) coupling said portion of said further vascular prosthesis with said main vascular prosthesis.

10. Method for realising a vascular prosthesis (10; 11) according to claim 9, **characterised in that** in said step (d), said portion of a further vascular prosthesis is fold and transversely sutured, so as to obtain said end portion chosen on the basis of one of the following groups:
- rectilinear portion;
- conical portion;
- rectilinear bevelled portion;
- conical bevelled portion.

11. Method for realising a vascular prosthesis (10; 11) according to one of preceding claims 9 - 10, **characterised in that** coupling in said steps (d) and/or /e) occurs by suture and/or gluing and/or welding, particularly heat welding.

12. Method for realising a vascular prosthesis (10; 11) according to one of preceding claims 9 - 11, **characterised in that** said main vascular prosthesis has elasticity along its longitudinal direction and said portion of said vascular prosthesis portion is obtained by a section of a segment of said main vascular prosthesis.

## Patentansprüche

1. Röhrenförmige Gefäßprothese (10; 11), die sich hauptsächlich entlang der Längsdimension erstreckt, wobei die Gefäßprothese (10; 11) umfasst
einen Hauptbereich (20; 21), der ein erstes (201; 201) und ein zweites Ende (202; 212) aufweist, und
mindestens einen Endbereich(30; 31), der sich entlang der Längsdimension erstreckt,
wobei der mindestens eine Endbereich (30; 31) mit einem von dem ersten (201; 211) oder zweiten Ende (202; 212) des Hauptbereichs (20; 21) gekoppelt ist und mit der Wand eines peripheren, venösen oder arteriellen Blutgefäßes gekoppelt werden kann und eine zylindrische oder kegelstumpfförmige Form aufweist,
so dass der mindestens eine Endbereich (30; 31) in der Lage ist, seinen Querschnitt an die wechselnde Bewegung anzupassen, die durch eine transversale oder radiale pulsierende Bewegung des Gefäßes erzeugt werden kann, mit dem der er koppelbar ist,
**dadurch gekennzeichnet, dass**
der mindestens eine Endbereich (30; 31) eine Elastizität entlang seiner transversalen Richtung in Bezug auf die Längsdimension aufweist und der Hauptbereich (20; 21) eine Elastizität entlang der Längsrichtung aufweist.

2. Gefäßprothese (10; 11) gemäß Anspruch 1, **dadurch gekennzeichnet, dass** sie zwei Endbereiche (30; 31) umfasst, die jeweils mit dem ersten (201; 211) und zweiten Ende (202; 212) gekoppelt sind.

3. Gefäßprothese (10; 11) gemäß einem der vorhergehenden Ansprüche, **dadurch gekennzeichnet, dass** der mindestens eine Endbereich (30; 31) und der Hauptbereich (20; 21) als ein einzelnes Stück realisiert sind.

4. Gefäßprothese (10; 11) gemäß einem der vorhergehenden Ansprüche 1-2 **dadurch gekennzeichnet, dass** der mindestens eine Endbereich (30; 31) mit dem relevanten Ende des Hauptbereichs (20; 21) für Nähen und/oder Kleben und/oder Schweißen, insbesondere Wärmeschweißen, gekoppelt ist.

5. Gefäßprothese (10; 11) gemäß einem der vorhergehenden Ansprüche 1-4, **dadurch gekennzeichnet, dass** der Hauptbereich (20; 21) eine transversale Elastizität in Bezug auf die Längsrichtung aufweist.

6. Gefäßprothese (10; 11) gemäß einem der vorhergehenden Ansprüche, **dadurch gekennzeichnet, dass** sie von dem Typ ist, der für einen peripheren Bypass und/oder Hämodialyse geeignet ist.

7. Gefäßprothese (10; 11) gemäß einem der vorhergehenden Ansprüche, **dadurch gekennzeichnet, dass** sie expandiertes Polytetrafluorethylen (PTFE), den Streck-, Standard- und/oder Intering-Typ und/oder Propaten-Typ und/oder jegliches anderes elastisches Prothesematerial umfasst.

8. Gefäßprothese (10; 11) gemäß einem der vorhergehenden Ansprüche, **dadurch gekennzeichnet, dass** der mindestens eine Endbereich (30; 31) auf der Grundlage der folgenden Gruppen gewählt ist:
- geradliniger Bereich;
- konischer Bereich;
- geradlinig angeschrägter Bereich;
- konisch angeschrägter Bereich.

9. Verfahren zur Realisierung einer Gefäßprothese (10; 11), wie sie in den Ansprüchen 1-8 definiert ist, umfassend die folgenden Schritte:
(a) Bereitstellung einer Haupt-Gefäßprothese;
(b) Bereitstellung eines Bereichs einer weiteren Gefäßprothese, die eine Elastizität entlang der Längsdimension fortlaufend über ihre Erstreckung aufweist;
(c) longitudinale Unterteilung des weiteren Bereichs der Gefäßprothese von Schritt (b);
(d) transversale Faltung und Kopplung des Bereichs der unterteilten Gefäßprothese von Schritt (c); und
(e) Kopplung des Bereichs der weiteren Gefäßprothese mit der Haupt-Gefäßprothese.

10. Verfahren zur Realisierung einer Gefäßprothese (10; 11) gemäß Anspruch 9, **dadurch gekennzeichnet, dass** bei Schritt (d) der Bereich einer weiteren Gefäßprothese gefaltet und transversal genäht wird, so dass der Endbereich erhalten wird, der auf der Basis von einer der folgenden Gruppen gewählt ist:
- geradliniger Bereich;
- konischer Bereich;
- geradlinig angeschrägter Bereich
- konisch angeschrägter Bereich.

11. Verfahren zur Realisierung einer Gefäßprothese (10; 11) gemäß einem der vorhergehenden Ansprüche 9-10, **dadurch gekennzeichnet, dass** die Kopplung in den Schritten (d) und/oder (e) durch Nähen und/oder Kleben und/oder Schweißen, insbesondere Wärmeschweißen, erfolgt.

12. Verfahren zur Realisierung einer Gefäßprothese (10; 11) gemäß einem der vorhergehenden Ansprüche 9-11, **dadurch gekennzeichnet, dass** die Haupt-Gefäßprothese eine Elastizität entlang ihrer Längsrichtung aufweist und der Bereich des Gefäßprothesenbereichs durch einen Abschnitt eines Segments der Haupt-Gefäßprothese erhalten wird.

## Revendications

1. Prothèse vasculaire de forme tubulaire (10 ; 11), s'étendant principalement le long de la dimension longitudinale, ladite prothèse vasculaire (10 ; 11) comprenant
une partie principale (20 ; 21), ayant une première (201 ; 201) et une seconde extrémité (202 ; 212), et
au moins une partie d'extrémité (30 ; 31) s'étendant le long de ladite dimension longitudinale
ladite au moins une partie d'extrémité (30 ; 31) est couplée à une de ladite première (201 ; 211) ou seconde extrémité (202 ; 212) de ladite partie principale (20 ; 21), et peut être couplée à la paroi d'un vaisseau sanguin veineux ou artériel périphérique, et a une forme cylindrique ou tronconique,
de sorte que ladite au moins une partie d'extrémité (30 ; 31) est susceptible de conformer sa section transversale au mouvement alterné qui peut être créé par un mouvement pulsatoire transversal ou radial dudit vaisseau auquel elle peut être couplée, **caractérisée en ce que** ladite au moins une partie d'extrémité (30 ; 31) a une certaine élasticité le long de sa direction transversale par rapport à ladite dimension longitudinale et ladite partie principale (20 ; 21) a une certaine élasticité le long de ladite direction longitudinale.

2. Prothèse vasculaire (10 ; 11) selon la revendication 1, **caractérisée en ce qu'**elle comprend deux parties d'extrémité (30 ; 31) respectivement couplées auxdites première (201 ; 211) et seconde extrémité (202 ; 212).

3. Prothèse vasculaire (10 ; 11) selon l'une des revendications précédentes, **caractérisée en ce que** ladite au moins une partie d'extrémité (30 ; 31) et ladite partie principale (20 ; 21) sont réalisées comme une pièce unique.

4. Prothèse vasculaire (10 ; 11) selon l'une des revendications précédentes 1 et 2, **caractérisée en ce que** ladite au moins une partie d'extrémité (30 ; 31) est reliée à l'extrémité appropriée de ladite partie principale (20 ; 21) pour suture et/ou collage et/ou soudage, particulièrement soudage à la chaleur.

5. Prothèse vasculaire (10 ; 11) selon l'une des revendications précédentes 1 à 4, **caractérisée en ce que** ladite partie principale (20 ; 21) a une élasticité transversale par rapport à ladite direction longitudinale.

6. Prothèse vasculaire (10 ; 11) selon l'une des revendications précédentes, **caractérisée en ce que** ladite prothèse est du type approprié pour un contournement périphérique et/ou une hémodialyse.

7. Prothèse vasculaire (10 ; 11) selon l'une des revendications précédentes, **caractérisée en ce que** ladite prothèse est composée de polytétrafluoréthylène expansé (PTFE), du type à étirement, standard et/ou fritté et/ou Propaten et/ou de toute autre matière prothétique élastique.

8. Prothèse vasculaire (10 ; 11) selon l'une des revendications précédentes, **caractérisée en ce que** ladite au moins une partie d'extrémité (30 ; 31) est choisie sur la base des groupes suivants :
- partie rectiligne ;
- partie conique ;
- partie biseautée rectiligne ;
- partie biseautée conique.

9. Procédé pour réaliser une prothèse vasculaire (10 ; 11) selon les revendications 1 à 8, comprenant les étapes suivantes :
(a) fourniture d'une prothèse vasculaire principale ;
(b) fourniture d'une partie d'une prothèse vasculaire supplémentaire ayant une certaine élasticité le long de ladite dimension longitudinale tout au long de son extension ;
(c) sectionnement longitudinal de ladite partie supplémentaire de prothèse vasculaire de ladite étape (b) ;
(d) pliage transversal et accouplement de ladite partie de prothèse vasculaire sectionnée de ladite étape (c) ; et
(e) accouplement de ladite partie de ladite prothèse vasculaire supplémentaire avec ladite prothèse vasculaire principale.

10. Procédé pour réaliser une prothèse vasculaire (10 ; 11) selon la revendication 9, **caractérisé en ce que**, dans ladite étape (d), ladite partie d'une prothèse vasculaire supplémentaire est pliée et suturée de façon transversale, afin d'obtenir ladite partie d'extrémité choisie sur la base d'un des groupes suivants :
- partie rectiligne ;
- partie conique ;
- partie biseautée rectiligne ;
- partie biseautée conique.

11. Procédé pour réaliser une prothèse vasculaire (10 ; 11) selon l'une des revendications précédentes 9 et 10, **caractérisé en ce que** l'accouplement dans lesdites étapes (d) et/ou (e) se produit par suture et/ou collage et/ou soudage, particulièrement soudage à la chaleur.

12. Procédé pour réaliser une prothèse vasculaire (10 ; 11) selon une des revendications précédentes 9 à 11, **caractérisé en ce que** ladite prothèse vasculaire principale a une certaine élasticité le long de sa direction longitudinale et ladite partie de ladite partie de prothèse vasculaire est obtenue par une section d'un segment de ladite prothèse vasculaire principale.
